# EUROPEAN PATENT SPECIFICATION

(11) **EP 1 610 854 B1**
(45) Date of publication and mention of the grant of the patent: **20.08.2008**
(21) Application number: 04715759.9
(22) Date of filing: 27.02.2004
(51) Int. Cl.: A61M 25/00, A61M 25/06, A61M 5/162, A61M 5/00

(54) **SAFETY NEEDLE AND CATHETER ASSEMBLY**
SICHERHEITSNADEL UND KATHETERANORDNUNG
ENSEMBLE D'AIGUILLE ET DE CATHETER DE SECURITE

(30) Priority: 08.04.2003 US 461172 P
(43) Date of publication of application: 04.01.2006
(73) Proprietor: Medex, Inc., Carlsbad, California 92008 (US)
(72) Inventor: McGURK, Joseph, P., Mason, OH 45040 (US)
(74) Representative: Findlay, Alice Rosemary
(86) International application number: PCT/US2004/006017
(87) International publication number: WO 2004/093961

(56) References cited:
- EP-A- 0 747 086
- EP-A- 1 112 754
- WO-A-94/23778
- WO-A-99/08742
- US-A- 4 013 080
- US-A- 5 120 321
- US-A1- 2002 177 818
- US-B1- 6 402 734

## Description

### Field of the Invention

The present invention relates, in general, to intravenous (IV) catheters and, more particularly, to a safety IV catheter with a needle tip protector that will automatically cover the needle tip upon needle withdrawal.

### Background of the Invention

An intravenous (IV) catheter is an instrument that is used to introduce certain fluids such as saline solution directly into the bloodstream of a patient. Typically, a needle or other stylet is first introduced through the cannula portion of the catheter and into the skin of the patient at the desired location such as the back of the patient's hand or a vessel on the inside of the arm. Once insertion is complete, the needle is removed from the cannula portion of the catheter. After removing the needle, a fluid handling device such as a syringe is attached to the luer fitting located at the proximal end of the catheter hub. Fluid then flows directly from the fluid handling device through the catheter into the bloodstream of the patient.

When the needle is removed from the cannula, the health care worker must place the exposed needle tip at a nearby location while simultaneously addressing the task required to accomplish the needle removal. It is at this juncture that the exposed needle tip creates a danger of an accidental needle stick occurring which leaves the health care worker vulnerable to the transmission of various, dangerous blood-borne pathogens such as human immune virus (HIV) and hepatitis.

The risk of a contaminated needle stick is not just isolated to the health care worker inserting the intravenous catheter. Careless disposal of used needles can put other health care workers at risk as well. Even others outside the health care profession, for example those involved in the clean-up and final disposal of medical waste, are at risk of an accidental needle stick from a carelessly discarded needle.

The danger to health care workers and others outside the health care profession from accidental needle sticks has yielded the development of catheters with safety mechanisms in which the occurrence of such accidental needle sticks is prevented. An example of a catheter having a safety mechanism is disclosed in US Patent No. Re. 34,416 issued to Lemieux. A safety catheter is described which includes an element that covers the needle tip upon removal of the needle from the catheter. The safety element includes a split flange at its proximal end which is expanded by the needle as the needle is inserted into an undersized hole at the center of this flange. The safety element is thus held secure within the catheter hub by inserting the needle through the undersized hole which forces the outside perimeter of the split flange against the inside wall of the catheter hub. One of the drawbacks to this design is the amount of friction force exerted against the needle by the split flange. A tight fit of the flange against the catheter wall causes great friction against the needle making it difficult to be withdrawn from the catheter by the clinician. A lose fit leaves the flange prone to releasing prematurely from the catheter as the needle is withdrawn, creating the potential that the needle tip will be left exposed.

Another example of a catheter having a safety mechanism is disclosed in US Patent No. 6,117,108 issued to Woehr et al. A safety IV catheter is described including a resilient needle guard which protects the needle tip upon removal of the needle from the catheter hub. The needle guard includes an arm that includes an opening through which a needle passes causing axial movement of the arm. This axial movement forces the arm into a groove or behind a rib located on the inside of the catheter hub, capturing the needle guard in the catheter hub. A potential issue with this design develops when the needle guard is not properly seated into the catheter hub. If the distal end of the needle guard arm is not in alignment with the groove in the catheter hub, excessive forces are placed on the needle causing a high drag force as the clinician removes the needle. And, since the needle guard arm is not properly seated in the groove, it may prematurely release from the catheter hub upon the removal of the needle leaving the needle tip exposed.

The prior art safety catheters all exhibit one or more drawbacks that have thus far limited their usefulness and full acceptance by health-care workers. What is needed therefore is a safety IV catheter that functions reliably, is easy and inexpensive to manufacture, and easy to use.

### Summary of the Invention

The closest prior art WO 99/08742 discloses a catheter introducer assembly comprising
a) a needle assembly comprising a needle having a diameter, a proximal end attached to a needle hub, a distal end extending therefrom, and a longitudinal axis therebetween,
b) a catheter assembly comprising a tubular catheter having a proximal end attached to a catheter hub, and a distal end, said needle being coaxially received within said catheter,
c) a needle tip protector disposed within said catheter hub, said needle tip protector being slidably disposed onto said needle, whereby when said needle is proximally removed from said catheter, said protector remains attached to said needle.

In accordance with the present invention there is provided a catheter introducer assembly which is characterised in that said needle further includes a bent area disposed between said proximal and distal ends, the bent area being such that a longitudinal axis of said distal end of said needle is off set from a longitudinal axis of said proximal end of said needle, and in that said needle tip protector is slidably disposed about said needle such that when said needle is proximally removed from said catheter said bent area prevents said needle tip protector from sliding off said needle so that said needle tip protector remains attached to said needle.

### Brief Description of the Drawings

The novel features of the invention are set forth with particularity in the appended claims. The invention itself, however, both as to organization and methods of operation, together with further objects and advantages thereof, may best be understood by reference to the following description, taken in conjunction with the accompanying drawings in which:
Figure 1 is a perspective view of the catheter and needle assembly of the present invention;
Figure 2 is an exploded perspective view of the catheter assembly and needle assembly including the needle tip protector of the present invention;
Figure 3 is a perspective view of the needle tip protector of the present invention;
Figure 4 is an elevation view of Figure 3 taken along line 4 - 4 illustrating the hole positions in the rear flanges of the needle tip protector as manufactured;
Figure 5 is a section view of the catheter assembly and needle assembly taken along line 5 - 5 of Figure 1;
Figure 6 is an enlarged partial section view of Figure 5 illustrating the relative position of the needle tip protector tab and catheter hub rib;
Figure 7 is a section view of the catheter hub with needle tip protector installed taken along line 7 - 7 of Figure 5;
Figure 8 is a perspective view of the needle tip protector shown as installed in the catheter hub with the needle inserted therethrough;
Figure 9 is a perspective view of the needle tip protector shown as removed from the catheter hub and illustrating the needle tip covered by the protector,
Figure 10 is a perspective view of an alternate embodiment of the needle tip protector of the present invention.

### Detailed Description of the Invention

As used herein, the term "proximal" refers to a location on the catheter and needle assembly with needle tip protector closest to the clinician using the device and thus furthest from the patient on which the device is used. Conversely, the term "distal" refers to a location farthest from the clinician and closest to the patient.

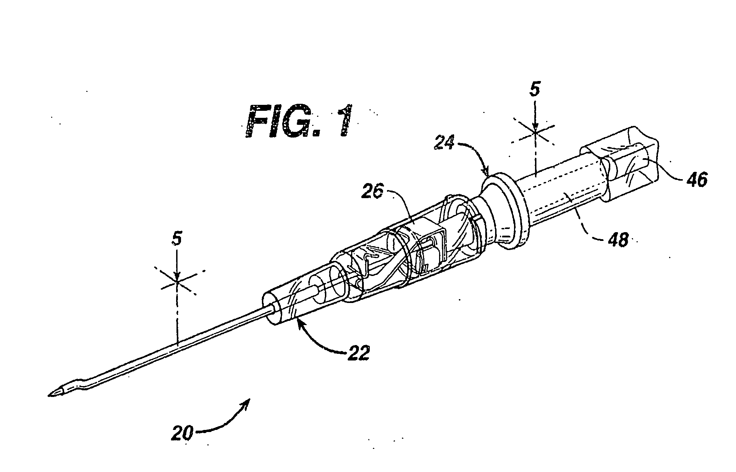

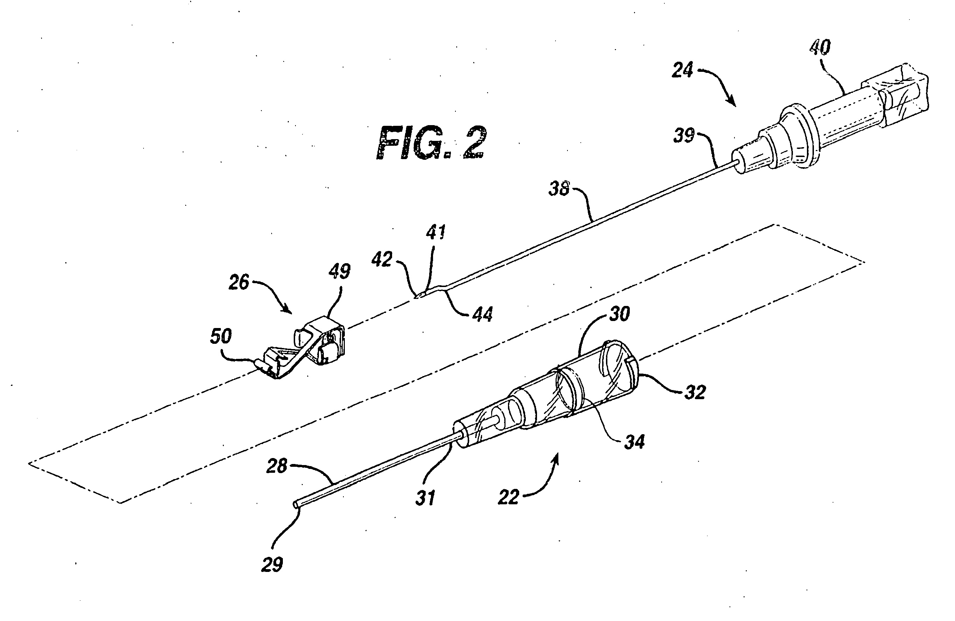

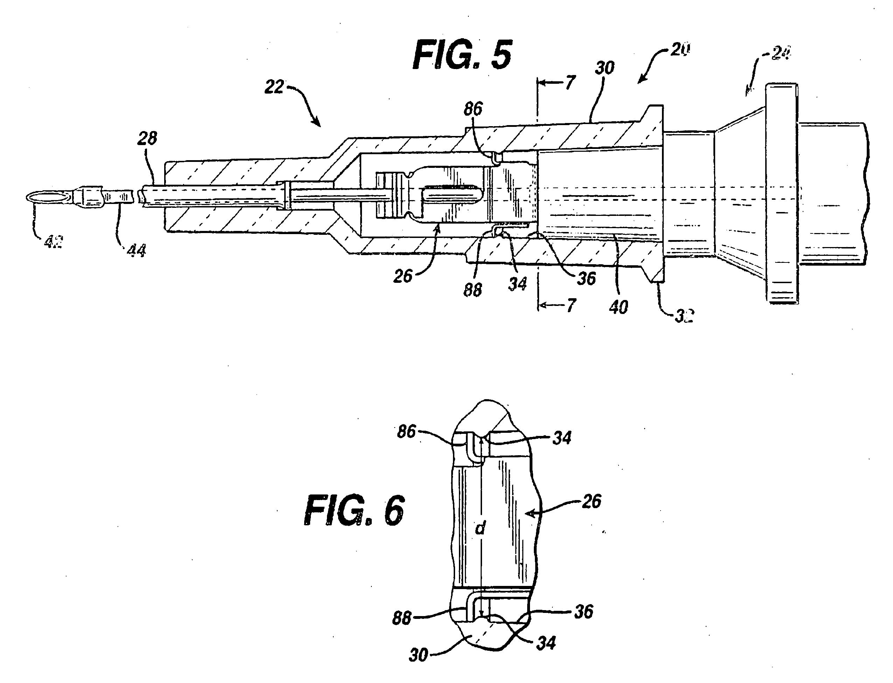

As illustrated in Figures 1 and 2, IV catheter assembly 20 comprises catheter assembly 22 and needle assembly 24. Needle assembly 24 further includes needle tip protector 26. Catheter assembly 22 includes catheter 28 which is a tubular structure having a proximal end 31 and distal end 29. Proximal end 31 of catheter 28 is fixedly attached to catheter hub 30. Catheters are well known in the medical art and one of many suitable materials, most of which are flexible thermoplastics, may be selected for use in catheter 28. Such materials may include, for example, polyurethane or fluorinated ethylene propylene. Catheter hub 30 is a generally tubular structure having an internal cavity in fluid communication with the internal lumen of catheter 28. Catheter hub 30 may be made from a suitable, rigid medical grade thermoplastic such as, for example, polypropylene or polycarbonate. For illustration purposes catheter hub 30 is shown translucent, though in actual use it may be translucent or opaque. At the proximal end of catheter hub 30 is integrally attached Luer fitting 32, commonly known in the medical art. Luer fitting 32 provides for secure, leakproof attachment of tubing, syringes, or any of many other medical devices used to infuse or withdraw fluids through the catheter assembly. As is more clearly illustrated in Figures 5 and 6, rib 34 is a raised annular ring integral to and extending from internal sidewall 36 of catheter hub 30. Rib 34 is located approximately mid way between the proximal end and distal end of sidewall 36. Rib 34 plays an important role in securing needle tip protector 26 in catheter hub 30, as will be described in more detail later.

Referring again to Figures 1 and 2, needle assembly 24 comprises needle 38, which is a tubular structure with a proximal end 39 and distal end 41, needle hub 40, and needle tip protector 26. Needle tip protector 26 is assembled slidably on needle 38. Needle 38 is preferably made of stainless steel. Proximal end 39 of needle 38 is fixedly attached to needle hub 40. A bevel 42 is located at the most distal end of needle 38 creating a sharp piercing tip. Needle bend 44 is located at the distal end of needle 38 proximal to bevel 42 and is curve in needle 38. Needle bend 44 can be created by bending method such as "rotary draw bending." Rotary draw bending is a process well known in the metal bending art and involves using a clamp die and a bend die, which are molded to match the nominal diameter of needle 38, to rotate needle 38 against a pressure die. A mandrel is inserted into needle 38 during the procedure to minimize the stretching that occurs along the outer radius of needle 38, while a wiper die reduces wrinkling along the inner radius of needle 38. Bend 44 is formed at an angle away from second flange hole 72 in needle tip protector 26 and is important in preventing the complete removal of needle tip protector 26 from needle 38, as will be described in more detail later. In the preferred embodiment the angle of bend 44 is 45 to 90 degrees away from second flange hole 72.

Needle hub 40 is generally a tubular structure having an internal cavity in fluid communication with the lumen in needle 38. It is preferably made of a translucent or transparent generally rigid thermoplastic material such as, for example, polycarbonate. At the most proximal end of the internal cavity in needle hub 40 is fixedly attached porous plug 46. A flashback chamber 48 is created in the cavity distal to porous plug 46. Porous plug 46 contains a plurality of microscopic openings which are large enough to permit the passage of air and other gasses but small enough to prevent the passage of blood. Flashback chamber 48 fills with blood upon successful entry of the needle tip into the targeted vein, providing the clinician visual conformation of the correct placement of the needle.

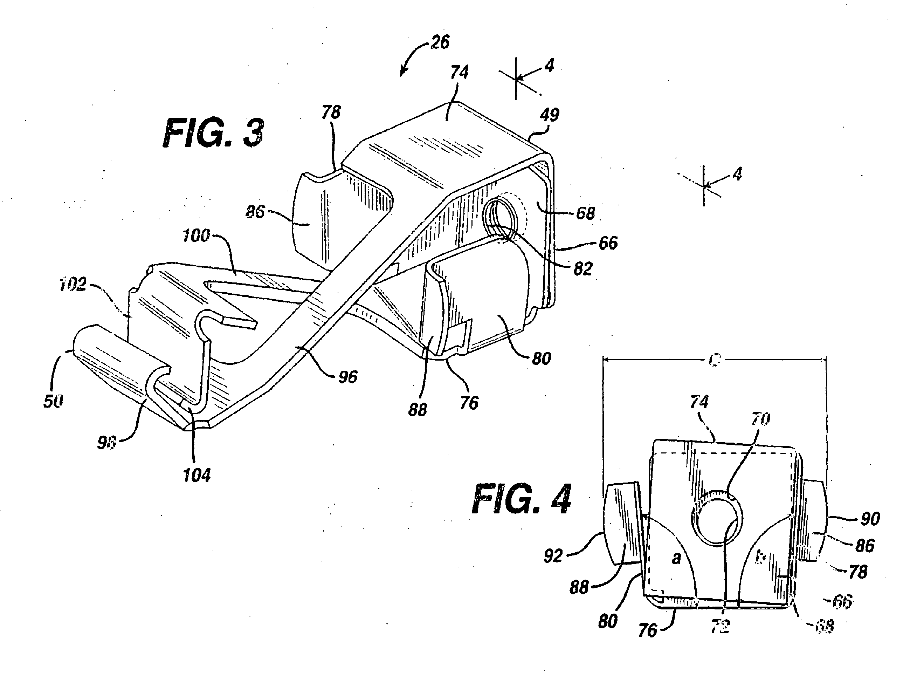

Referring now to Figures 3 and 4, needle tip protector 26 has a proximal end 49 and distal end 50 and is preferably a unitary structure formed from a single piece of thin, resilient material, preferably stainless steel. First flange 66 and second flange 68 are generally square and are integrally connected at right angles to first outer wall 74 and second outer wall 76, respectively. First outer wall 74 is connected at a right angle to first tab flange 78. First tab flange 78 and second tab flange 80 are each formed at angles slightly greater than 90 degrees to second outer wall 76 so that the resulting dimension c is slightly larger than inside diameter d (see Figures 5 - 7) across rib 34 in catheter hub 30. In the preferred embodiment angles a and b are each approximately 94.25 degrees. In the preferred embodiment dimension c is approximately .005 - .009 inches larger than dimension d. First flange hole 70 is located in the center of first flange 66 and is over-sized to slidably receive needle 38. Second flange hole 72 and skirt 82 are located in the center of second flange 68. Skirt 82 is integral to second flange hole 72 and is formed by extruding material from second flange hole 72 in a direction distal to second flange 68. This permits for a very close but slidably fit over the nominal diameter of needle 38. Skirt 82 also functions to help maintain alignment of needle 38 to the center axis of needle tip protector 26. As would be understood by one skilled in the art, flange hole 72 would be appropriately sized to the particular needle "gauge" size to which it is designed to receive. First tab 86 and second tab 88 are connected at right angles to first tab flange 78 and second tab flange 80, respectively, and protrude outward away from the center axis of needle tip protector 26. First tab edge 90, located on the outer portion of first tab 86, and second tab edge 92, located on the outer portion of second tab 88, are each arcuate to approximately match the curve of sidewall 36 in catheter hub 30.

Referring again to Figure 3, first beam 96 extends distally from first outer wall 74 and is angled toward and extends past the center axis of needle tip protector 26. At the distal end of first beam 96 is integrally formed curved first lip 98 which extends across and through the center axis of needle tip protector 26. Second beam 100 extends distally from second outer wall 76 and is angled toward and extends past the center axis of needle tip protector 26. At the distal end of second beam 100 is stop flange 102 which extends across and normal to the center axis of needle tip protector 26. At the end of stop flange 102 opposite its connection to second beam 100 is integrally formed curved second lip 104.

Referring now to Figures 5-9, needle tip protector 26 is assembled to needle 38 as follows;

The proximal end of needle 38 is fixedly attached to the distal end of needle hub 40, which contains porous plug 46 fixedly attached to its proximal end;

The distal end of needle 38 is inserted through first flange hole 70 and then through second flange hole 72 in needle tip protector 26, moving from proximal to distal;

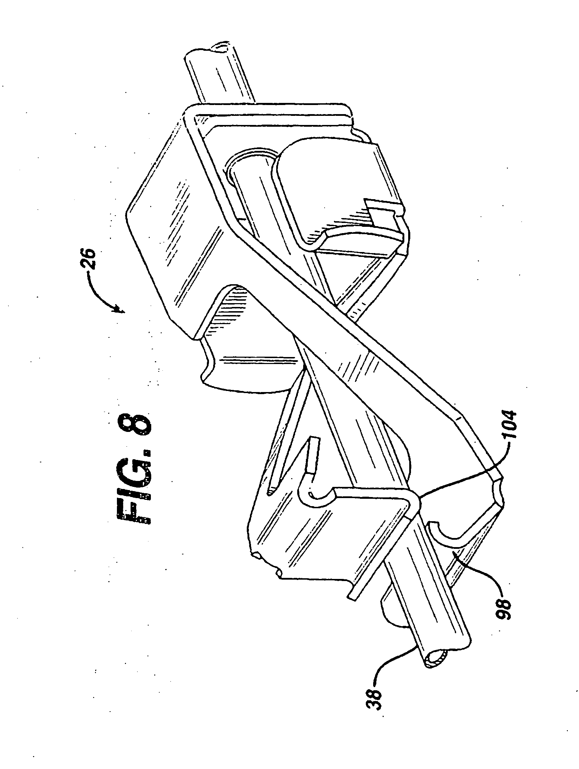

First beam 96 and second beam 100 are flexed, as a result of their resilient properties, normal to the center axis of needle tip protector 26 so that needle 38 will pass between first lip 98 and second lip 104 (see Figure 8);

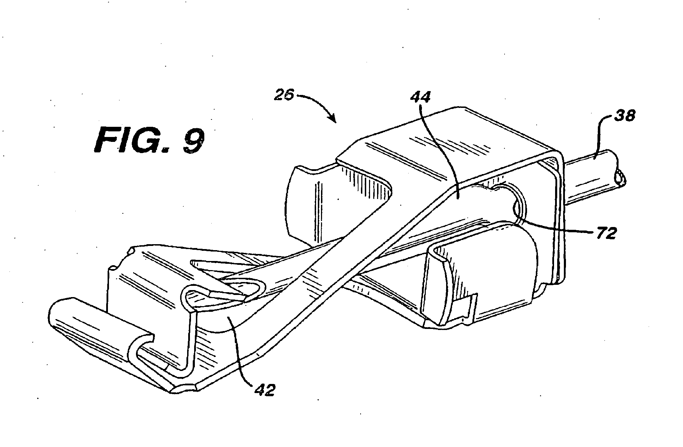

Needle bend 44 is added to the distal end of needle 38 just proximal to bevel 42. Bend 44 is angled away from second flange hole 72 locally (see Figure 9) thus preventing the complete removal of needle tip protector 26 from the distal end of needle 38.

Now needle assembly 24, including needle tip protector 26, is assembled into catheter assembly 22 as follows;

The distal end of needle 38 is positioned into the proximal end of catheter hub 30 and needle assembly 24 is moved distally causing needle 38 to enter catheter 28;

As needle assembly 24 continues to move distally, needle tip protector 26 enters the opening in the proximal end of catheter hub 30;

Continued distal movement of needle assembly 24 causes the distal edge of needle hub 40 to push first tab 86 and second tab 88 on needle tip protector 26 into contact with rib 34 located on hub sidewall 36;

Continued distal movement forces first tab 86 and second tab 88, due to the resilient properties of needle tip protector 26, past rib 34 and in contact with sidewall 36, just distal to rib 34.

Needle tip protector 26 is thus held distal to rib 34 inside the cavity in catheter hub 30 by the flexural forces of first tab 86 and second tab 88 since dimension c on needle tip protector 26 is larger than dimension d across rib 34 inside catheter hub 30. (see Figure 6).

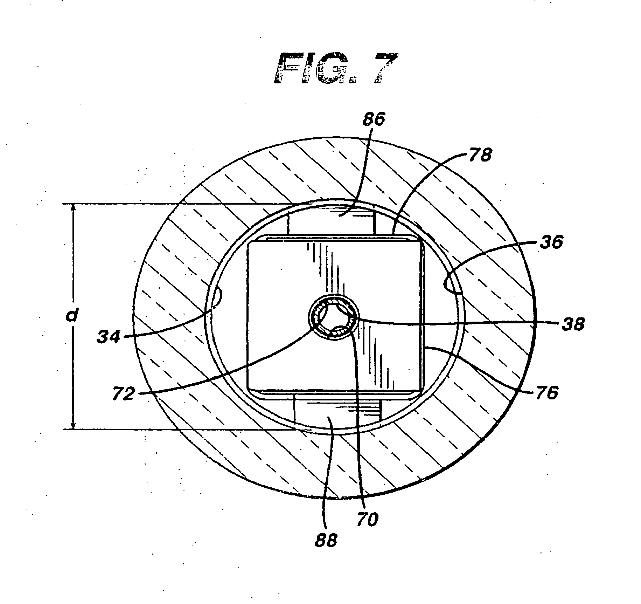

As is best illustrated in Figure 7, the movement of first tab 86 and second tab 88 as needle tip protector 26 is finally seated distal to rib 34 causes flexure in second outer wall 76 and first tab flange 78 resulting in the approximate alignment of first flange hole 70 and second flange hole 72.

Now, in actual clinical use, the IV catheter assembly 20 of the present invention functions as follows;

The distal end of needle 38, which extends just past the distal end of catheter 28 is inserted into the patient's vein;

The clinician observes blood in the flash chamber in needle hub 40;

The clinician grasps needle hub 40, and catheter assembly 22 alone is moved distally into the vein;

The clinician applies slight pressure to the insertion site to hold catheter assembly 22 secure;

The clinician grasps the needle hub and begins withdrawal of needle assembly 24 from catheter assembly 22. During this process, needle tip protector 26 remains secure inside catheter hub 30 until bend 44 on the distal end of needle 38 comes into contact with second flange hole 72. Just before bend 44 encounters second flange hole 72, the biasing forces of first beam 96 and second beam 100 cause stop flange 102 and first lip 98 to move normal to and across the center axis of needle 38, blocking any further distal movement of needle 38 relative to needle tip protector 26. After stop flange 102 and first lip 98 move normal to and across the center axis of needle 38, first beam 96 and second beam 100 prevent axial movement of needle 38 preventing needle 38 from being twisted enabling bend 44 to be manipulated through second flange hole 72;

Since bend 44 is angled away from second flange hole 72 and first beam 96 and second beam 100 prevent axial movement of needle 38 securing bend 44 from being manipulated through second flange hole 72, continued proximal movement of needle 38 carries needle tip protector 26 proximal as well, forcing first tab 86 and second tab 88 on needle tip protector 26 against rib 34. First tab 86 and second tab 88 are forced to flex normal to and toward the center axis of needle tip protector 26, permitting continued movement proximal, past rib 34;

Needle assembly 24 is now removed entirely from catheter assembly 22, with the needle tip covered by needle tip protector 26 of the present invention.

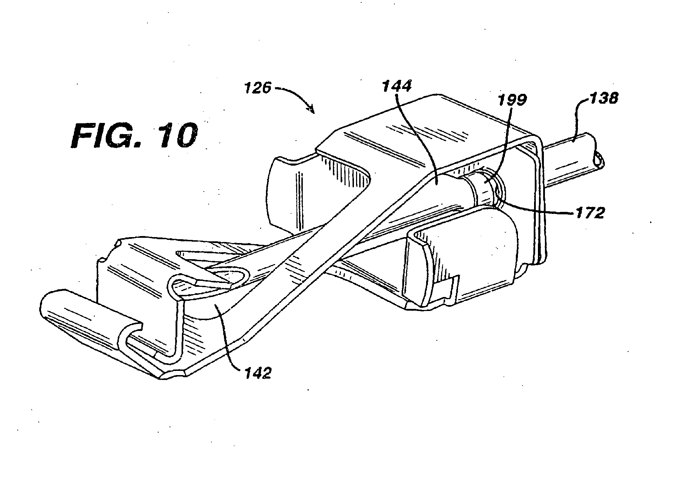

Figure 10 shows an alternate embodiment of the present invention. In this embodiment, needle 138, similar to needle 38, includes sleeve 199. Sleeve 199 can be slidably or fixedly attached on needle 138 proximal to bend 144 such that sleeve 199 is biased against second flange hole 172 when needle 138 is removed from catheter assembly 22. Sleeve 199 helps prevent needle tip protector 126 from being completely removed from needle 138 by ensuring that bend 144 can not be manipulated through second flange hole 172.

While preferred embodiments of the present invention have been shown and described herein, it will be obvious to those skilled in the art that such embodiments are provided by way of example only. Numerous variations, changes, and substitutions will now occur to those skilled in the art without departing from the invention. Accordingly, it is intended that the invention be limited only by the scope of the appended claims. In addition, it should be understood that every structure described above has a function and such structure can be referred to as a means for performing that function.

## Claims

1. A catheter introducer assembly, comprising
a) a needle assembly (24) comprising a needle (38, 138) having a diameter, a proximal end (39) attached to a needle hub (40), a distal end (41) extending therefrom, and a longitudinal axis therebetween,
b) a catheter assembly (22) comprising a tubular catheter (28) having a proximal end (31) attached to a catheter hub (30), and a distal end, said needle (38, 138) being coaxially received within said catheter (28),
c) a needle tip protector (26, 126) disposed within said catheter hub (30), said needle tip protector (26, 126) being slidably disposed onto said needle (38, 138), whereby when said needle (38, 138) is proximally removed from said catheter (28), said protector (26, 126) remains attached to said needle (38, 138),
**characterised in that** said needle (38, 138) further includes a bent area (44, 144) disposed between said proximal and distal ends (39, 40), the bent area (44, 144) being such that a longitudinal axis of said distal end (41) of said needle (38, 138) is off set from a longitudinal axis of said proximal end (39) of said needle, and **in that** said needle tip protector (26, 126) is slidably disposed about said needle (38, 138) such that when said needle (38, 138) is proximally removed from said catheter (28) said bent area (44, 144) prevents said needle tip protector (26, 126) from sliding off said needle (38, 138) so that said needle tip protector (26, 126) remains attached to said needle (38, 138).

2. The catheter introducer assembly of claim 1 wherein said bent area (44, 144) on said needle is angled 45-90 degrees away from its original direction.

3. The catheter introducer assembly of either claim 1 or claim 2 wherein said needle diameter is constant between its distal and proximal ends (39, 40).

4. The catheter introducer assembly of any preceding claim wherein said needle (138) further comprises of a sleeve (199).

5. The catheter introducer assembly of claim 4 wherein said sleeve (199) is attached proximal to said bent area (144) to help secure said needle (138) in said protector (126) when said needle (138) is removed from said catheter (28).

6. The catheter introducer assembly of any preceding claim wherein said needle (38, 138) is hollow.

## Patentansprüche

1. Kathetereinführungsbaugruppe, die Folgendes umfasst:
a) eine Kanülenbaugruppe (24), die eine Kanüle (38, 138) mit einem Durchmesser, einem proximalen Ende (39), das an einer Kanülennabe (40) angebracht ist, einem distalen Ende (41), das sich davon erstreckt, und eine Längsachse dazwischen umfasst,
b) eine Katheterbaugruppe (22), die einen röhrenförmigen Katheter (28) mit einem proximalen Ende (31), das an einer Katheternabe (30) angebracht ist, und einem distalen Ende umfasst, wobei die genannte Kanüle (38, 138) koaxial in dem genannten Katheter (28) aufgenommen wird,
c) eine Kanülenspitzenschutzvorrichtung (26, 126), die sich innerhalb der genannten Katheternabe (30) befindet, wobei die genannte Kanülenspitzenschutzvorrichtung (26, 126) gleitfähig auf der genannten Kanüle (38, 138) angeordnet ist, wobei, wenn die genannte Kanüle (38, 138) proximal von dem genannten Katheter (28) entfernt wird, die genannte Schutzvorrichtung (26, 126) an der genannten Kanüle (38, 138) angebracht bleibt,
**dadurch gekennzeichnet, dass** die genannte Kanüle (38, 138) ferner einen gekrümmten Bereich (44, 144) zwischen dem genannten proximalen und distalen Ende (38, 40) aufweist, wobei der gekrümmte Bereich (44, 144) derart ist, dass eine Längsachse des genannten distalen Endes (41) der genannten Kanüle (38, 138) von einer Längsachse des genannten proximalen Endes (39) der genannten Kanüle versetzt ist, und **dadurch**, dass die genannte Kanülenspitzenschutzvorrichtung (26, 126) gleitfähig um die genannte Kanüle (38, 138) herum angeordnet ist, so dass, wenn die genannte Kanüle (38, 138) proximal von dem genannten Katheter (28) entfernt wird, der genannte gekrümmte Bereich (44, 144) verhindert, dass die genannte Kanülenspitzenschutzvorrichtung (26, 126) von der genannten Kanüle (38, 138) herunter gleitet, so dass die genannte Kanülenspitzenschutzvorrichtung (26, 126) an der genannten Kanüle (38, 138) angebracht bleibt.

2. Kathetereinführungsbaugruppe nach Anspruch 1, wobei der genannte gekrümmte Bereich (44, 144) auf der genannten Kanüle 45-90 Grad von der ursprünglichen Richtung weg abgewinkelt ist.

3. Kathetereinführungsbaugruppe nach Anspruch 1 oder Anspruch 2, wobei der genannte Kanülendurchmesser zwischen dem distalen und proximalen Ende (39, 40) konstant ist.

4. Kathetereinführungsbaugruppe nach einem der vorherigen Ansprüche, wobei die genannte Kanüle (138) ferner eine Hülse (199) umfasst.

5. Kathetereinführungsbaugruppe nach Anspruch 4, wobei die genannte Hülse (199) proximal an dem genannten gekrümmten Bereich (144) angebracht ist, um beim Sichern der genannten Kanüle (138) in der genannten Schutzvorrichtung (126) behilflich zu sein, wenn die genannte Kanüle (138) von dem genannten Katheter (28) entfernt wird.

6. Kathetereinführungsbaugruppe nach einem der vorherigen Ansprüche, wobei die genannte Kanüle (38, 138) hohl ist.

## Revendications

1. Ensemble d'introduction de cathéter, comprenant
a) un ensemble d'aiguille (24) comprenant une aiguille (38, 138) ayant un diamètre, une extrémité proximale (39) fixée à une embase d'aiguille (40), une extrémité distale (41) s'étendant à partir de cette extrémité proximale, et un axe longitudinal entre elles,
b) un ensemble de cathéter (22) comprenant un cathéter tubulaire (28) ayant une extrémité proximale (31) fixée à une embase de cathéter (30), et une extrémité distale, ladite aiguille (38, 138) étant reçue coaxialement à l'intérieur dudit cathéter (28),
c) un protecteur de pointe d'aiguille (26, 126) disposé à l'intérieur de ladite embase de cathéter (30), ledit protecteur de pointe d'aiguille (26, 126) étant disposé de façon coulissante sur ladite aiguille (38, 138), de telle sorte que lorsque ladite aiguille (38, 138) est retirée proximalement dudit cathéter (28), ledit protecteur (26, 126) reste fixé à ladite aiguille (38, 138),
**caractérisé en ce que** ladite aiguille (38, 138) comporte en outre une partie coudée (44, 144) disposée entre lesdites extrémités proximale et distale (39, 40), la partie coudée (44, 144) étant telle qu'un axe longitudinal de ladite extrémité distale (41) de ladite aiguille (38, 138) est décalé par rapport à un axe longitudinal de ladite extrémité proximale (39) de ladite aiguille, et **en ce que** ledit protecteur de pointe d'aiguille (26, 126) est disposé de façon coulissante autour de ladite aiguille (38, 138) de telle sorte que lorsque ladite aiguille (38, 138) est retirée proximalement dudit cathéter (28) ladite partie coudée (44, 144) empêche ledit protecteur de pointe d'aiguille (26, 126) de glisser et de se séparer de ladite aiguille (38, 138) si bien que ledit protecteur de pointe d'aiguille (26, 126) reste fixé à ladite aiguille (38, 138).

2. Ensemble d'introduction de cathéter selon la revendication 1, dans lequel ladite partie coudée (44, 144) sur ladite aiguille est écartée de sa direction d'origine selon un angle d'inclinaison de 45 à 90 degrés.

3. Ensemble d'introduction de cathéter selon la revendication 1 ou 2, dans lequel ledit diamètre d'aiguille est constant entre ses extrémités distale et proximale (39, 40).

4. Ensemble d'introduction de cathéter selon l'une quelconque des revendications précédentes, dans lequel ladite aiguille (138) comprend en outre un manchon (199).

5. Ensemble d'introduction de cathéter selon la revendication 4, dans lequel ledit manchon (199) est fixé proximalement à ladite partie coudée (144) afin d'aider à fixer ladite aiguille dans ledit protecteur (126) quand ladite aiguille (138) est retirée dudit cathéter (28).

6. Ensemble d'introduction de cathéter selon la revendication précédente, dans lequel ladite aiguille (38, 138) est creuse.
